# EUROPÄISCHE PATENTSCHRIFT

(11) **EP 0 362 712 B1**
(45) Veröffentlichungstag und Bekanntmachung des Hinweises auf die Patenterteilung: **21.04.1993**
(21) Anmeldenummer: 89118075.4
(22) Anmeldetag: 29.09.1989
(51) Int. Cl.: A61M 35/00

(54) **Applikator zum Auftragen von flüssigen Mitteln auf die Haut**
Applicator for dispensing fluidic substances onto the skin
Applicateur pour distribuer les produits liquides sur la peau

(30) Priorität: 29.09.1988 DE 8812344 U
(43) Veröffentlichungstag der Anmeldung: 11.04.1990
(73) Patentinhaber: Lichtwer Pharma GmbH, D-13467 Berlin (DE)
(72) Erfinder: Rohmeder, Jürgen, Dr., W-8266 Töging/Inn (DE)
(74) Vertreter: Maikowski, Michael, Dipl.-Ing. Dr.

(56) Entgegenhaltungen:
- AU-B- 562 341
- CH-A- 217 080
- DE-U- 8 510 160
- FR-A- 984 176
- FR-E- 85 853

## Beschreibung

Die Erfindung betrifft einen Applikator nach dem Oberbegriff des Anspruchs 1.

Ein derartiger Applikator für flüssiges Lippenrot ist aus der CH-A-0217 080 bekannt, dessen Auftrageinrichtung einen Schaft aufweist. Im unteren Ende des am Gehäuseverschluß befestigten Schaftes ist eine Bohrung vorgesehen. In dieser Bohrung sitzt ein zylindrisches Auftragmittel aus einem Aufsaugmaterial, wie z. B. Schwammgummi, Watte, Filz, Kork, Pinsel und dergleichen. Dieses zylindrische Auftragmittel ragt in den Raum zwischen dem Schaft und dem Boden des Behälters hinein, reicht aber nicht bis zu diesem Boden hin. Bei diesem Applikator erstreckt sich nur ein kurzes Zylinderende des Auftragmittels aus der Bohrung heraus.

Dieses Zylinderende ist starr und kann ab einem bestimmten Füllungsgrad des Applikators nicht mehr benetzt werden. Für ein großflächiges Auftragen auf die Haut ist dieser Applikator nicht geeignet. Das starre Zylinderende ruft beim Auftragen auf eine empfindliche oder traumatischen Einwirkungen unterliegende Haut unerwünschte Affektionen hervor.

Der Erfindung liegt die Aufgabe zugrunde, einen Applikator zu schaffen, der zum großflächigen, druckfreien und raschen Auftragen eines flüssigen Mittels auf die menschliche Haut sofort verwendbar ist.

Erfindungsgemäß wird diese Aufgabe dadurch gelöst, daß die Auftrageinrichtung ein derart breites, dünnes Filzstückchen aufweist, daß dieses in auswechselbarer Weise mit einem Ende mit Haftreibung in einer Tasche sitzt, die den Querschnitt eines flachen Rechtecks aufweist und sich vom Verschlußdeckel senkrecht erstreckt, und daß dieses so elastisch ist, daß es ohne Ausübung eines unerwünschten Druckes auf die Haut über diese großflächig führbar ist, wobei das Filzstückchen eine solche Länge aufweist, daß es im geschlossenen Behälter bis zu dessen Boden reicht.

Mit besonderem Vorteil werden die Breiten- und Dickenabmessung des Filzstückchens so gewählt, daß eine Elastizität des Filzstückchens erzielt wird, die es ermöglicht, daß es ohne Ausübung eines unerwünschten Druckes auf die Haut über diese großflächig führbar ist. Für die Aufbringung eines Arzneimittels auf die Haut genügt es, mit dem Filzstückchen des Applikators leicht über die Haut zu streichen und die Haut lediglich zu berühren. Die Schmerz-Rezeptoren der Haut sprechen beispielsweise auf ein auf diese Weise aufgetragenes, in Wasser gelöstes Prokainhydrochlorid oder in Äthanol gelöstes Menthol bereits an und erzeugen eine Wirkung auf die Nervenenden, die eine vollständige oder teilweise Beseitigung der Schmerzempfindung bewirkt.

Dadurch, daß das Filzstückchen eine solche Länge aufweist, daß es im geschlossenen Behälter bis zu dessen Boden reicht, wird bis zum Verbrauch des Behälterinhaltes das Filzstückchen ständig gesättigt und ist durchfeuchtet. Eine derartige Sättigung und Durchfeuchtung ist aber zum Auftragen eines Mittels auf die menschliche Haut bei Anwendung eines möglichst geringen Berührungsdruckes notwendig.

Dadurch, daß dieses Filzstückchen in auswechselbarer Weise mit einem Ende mit Haftreibung in der Tasche sitzt, kann ein verschmutztes Filzstückchen leicht ausgewechselt werden. Eine wiederholte Anwendung des Applikators führt zu einer allmählichen Verunreinigung des Filzstückchens durch Hautfett. Die Haftreibung gewährleistet eine sichere Verwendung, da bei dieser Verwendung das Filzstückchen nicht aus der Tasche herausgezogen werden kann.

Da die Tasche in verschiedenen Anwendungsfällen eine wesentlich größere vertikale Erstreckung aufweisen kann als das Filzstückchen, sind mit Vorteil in der Tasche Einschubbegrenzungsrippen für das Filzstückchen vorgesehen.

Mit besonderem Vorteil erstreckt sich die Tasche in Durchmesserrichtung des Verschlußdeckels, so daß eine optimale Breitenabmessung des Filzstückchens erzielt werden kann.

Bei einer besonders vorteilhaften Ausführungsform weist das Filzstückchen eine Stärke von etwa 3 mm, eine Breite von etwa 20 mm und eine Höhe von etwa 20 mm auf.

Ein Ausführungsbeispiel der Erfindung wird nachfolgend anhand der Zeichnung erläutert. Es zeigen:

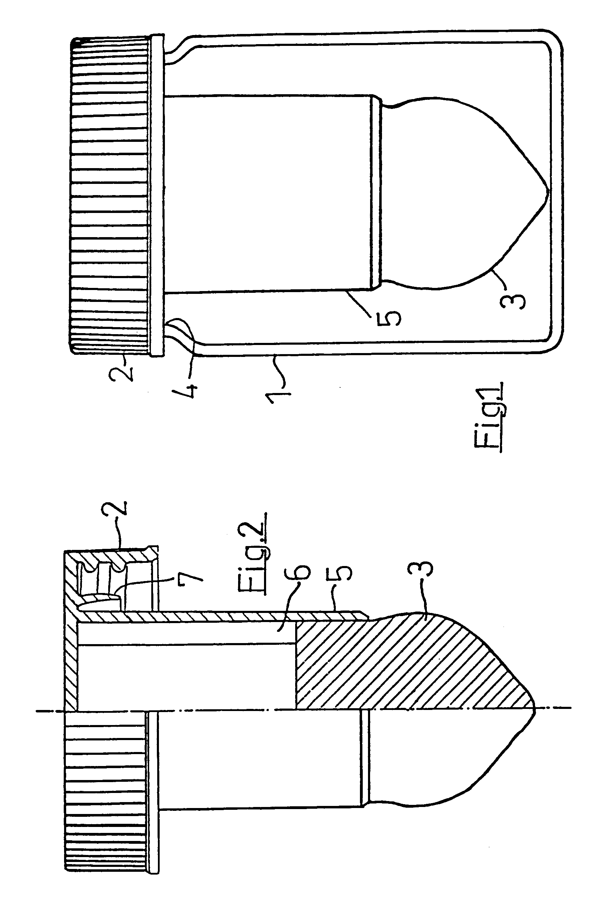

- Fig. 1: den Applikator in Seitenansicht im geschlossenen Zustand;
- Fig. 2: den Verschlußdeckel in Seitenansicht und teilweise im Schnitt und

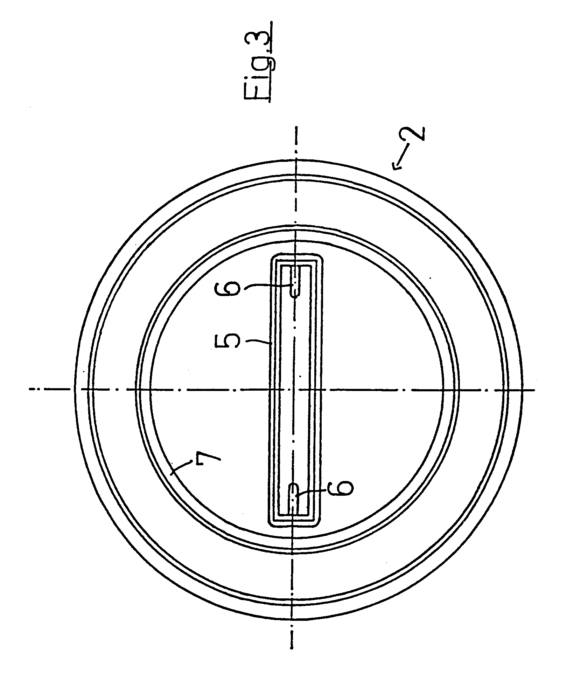

- Fig. 3: den Verschlußdeckel in einer Ansicht von unten.

Der dargestellte Applikator weist einen niedrigen, breiten Behälter 1 mit kreisförmigem, ovalem, drei- oder mehrkantigem Querschnitt auf, der das Körperpflegemittel oder dergleichen aufnimmt. Der Behälter 1 hat eine relativ grosse Öffnung 4 von etwa 4 cm Durchmesser, die durch einen Schraub-Verschlussdeckel 2 verschliessbar ist. Im Inneren des Verschlussdeckels 2 ist eine Dichtungslippe 7 und eine nach unten abstehende Tasche 5 angeformt, in der ein Filzstückchen 3 mit seinem einen Ende eingeschoben ist. Die Tasche steht von dem Verschlussdeckel 2 senkrecht nach unten ab und erstreckt sich in Durchmesserrichtung des Verschlussdeckels 2. Seitlich ist zwischen der Tasche 5 und dem Rand des Verschlussdeckels 2 ein ausreichender Raum für die Dichtungslippe 7 und den Öffnungsrand des Behälters 1. In Durchmesserrichtung ist die Abmessung der Tasche daher etwas kleiner als die Öffnung des Behälters 1. Um das Filzstückchen 3 sicher zu halten, genügt eine Tiefe der Tasche 5 von etwa 1 cm. Die Breite der Tasche 5 ist dabei so auf die Stärke des Filzstückchens 3 abgestimmt, dass das Filzstückchen 3 einerseits leicht in die Tasche 5 eingeschoben werden kann, andererseits darin ohne weitere Hilfsmittel ausreichend festgehalten wird, so dass es bei der Verwendung des Applikators nicht herausrutscht. Die Tasche 5 hat bei dem Ausführungsbeispiel zwar eine wesentliche größere, vertikale Erstreckung, Anschlagrippen 6 verhindern jedoch, daß das Filzstückchen 3 tiefer als etwa 1 cm in die Tasche 5 gesteckt werden kann. Die Anschlagrippen 6 sind in vertikaler Richtung in den Seiten der Tasche 5 angeordnet und enden in einem Abstand von etwa 1 cm von der unteren Öffnung der Tasche. Da das Filzstückchen 3 von Natur aus zusammendrückbar ist und eine rauhe Oberfläche hat, reicht die Reibung normalerweise bereits aus, um das Filzstückchen 3 genügend fest in der Tasche 5 zu halten.

Das Filzstückchen 3 hat eine solche Länge, dass es bei verschlossenem Behälter 1 bis zum Boden des Behälters 1 reicht. Dadurch wird erreicht, dass der gesamte Inhalt des flüssigen Arzneimittels von dem Filzstückchen 3 aufgenommen werden kann.

Das Filzstückchen 3 besteht aus einer regellosen Faseranordnung. Es eignen sich insbesondere Schafwolle, Baumwolle, Kuhhaare und Pflanzenfasern. Chemiefasern sind nur geeignet, wenn nicht die Gefahr der Herauslösung von Weichmachern oder sonstigen Zusatzstoffen besteht. Grundsätzlich sind alle Fasern geeignet, die die Flüssigkeiten beim Bestreichen der menschlichen Haut in einer Weise austreten lassen, dass die Flüssigkeiten auf der Haut gleichmässig verteilt werden, ohne die Haut mechanisch zu reizen, bis schließlich durch die natürliche Wärme der menschlichen Haut das Lösungsmittel langsam verdunstet.

Der erfindungsgemässe Applikator kann z.B. zum Auftragen einer Lösung aus 4 g Lidocainhydrochlorid und 96 g Wasser oder einer Lösung aus 10 g Pfefferminzöl, 5 g Menthol und 5 g Eukalyptusöl in 80 g 90 %igem Ethanol verwendet werden. Zur Linderung von hautnahen Schmerzen wird dieses Mittel in der Weise aufgetragen, dass der Verschlussdeckel 2 abgeschraubt wird und das in der Tasche 5 des Verschlussdeckels 2 gehaltene Filzstückchen 3 mit möglichst geringem Druck über die Stirne geführt wird, wobei das Filzstückchen 3 entgegen der Bewegungsrichtung zeigt. Wegen seiner geringen Stärke von nur etwa 3 mm ist das Filzstückchen 3 so elastisch, dass die Ausübung eines unerwünschten Druckes auf die schmerzempfindliche Region mit Sicherheit verhindert wird.

## Patentansprüche

1. Applikator zum Auftragen flüssiger Schönheits-, Körperpflege- oder Arzneimittel auf die Haut mit einem Behälter (1) und einem Verschlußdeckel (2) auf dessen Innenseite eine, bei verschlossenem Behälter in diesen hineinragende Auftrageinrichtung befestigt ist,
**dadurch gekennzeichnet,** daß
die Auftrageinrichtung ein derart breites, dünnes Filzstückchen (3) aufweist, daß diess
in auswechselbarer Weise mit einem Ende mit Haftreibung in einer Tasche (5) sitzt, die den Querschnitt eines flachen Rechtecks aufweist und sich vom Verschlußdeckel (2) senkrecht erstreckt,
und daß dieses
so elastisch ist, daß es ohne Ausübung eines unerwünschten Druckes auf die Haut über diese großflächig führbar ist, wobei das Filzstückchen eine solche Länge aufweist, daß es im geschlossenen Behälter (1) bis zu dessen Boden reicht.

2. Applikator nach Anspruch 1, **dadurch gekennzeichnet,** daß in der Tasche (5) Einschubbegrenzungsrippen (6) für das Filzstückchen (3) angeordnet sind.

3. Applikator nach Anspruch 1 oder 2, **dadurch gekennzeichnet,** daß sich die Tasche (5) in Durchmesserrichtung des Verschlußdeckels (2) erstreckt.

4. Applikator nah mindestens einem der Ansprüche 1 bis 3, **dadurch gekennzeichnet,** daß das Filzstückchen (3) eine Stärke von 3 mm, eine Breite von 20 mm und eine Höhe von 20 mm hat.

## Claims

1. Applicator for applying liquid beauty, body care or medicinal preparations to the skin, having a container (1) and a closure lid (2) on the inside of which is fixed an application device which when the container is closed projects into same,
characterised in that the application device has a thin felt member (3) which is so wide that it sits in a replaceably manner with one end with static friction in a pocket (5) which has the cross-section of a flat rectangle and extends perpendicularly from the closure lid (2),
and that this felt member is so elastic that without exerting any undesired pressure on the skin it can be moved with its wide surface over the skin whereby the length of the felt member is such that it reaches right down to the bottom of the container when the container (1) is closed.

2. Applicator according to claim 1 characterised in that ribs (6) are provided in the pocket (5) to restrict the insert depth of the felt member.

3. Applicator according to claim 1 to 2 characterised in that the pocket (5) extends in the diameter direction of the closure lid (2).

4. Applicator according to at least one of claims 1 to 3 characterised in that the felt member has a thickness of 3mm, a width of 20 mm and a height of 20 mm.

## Revendications

1. Applicateur destiné à l'application de produits liquides de beauté, de soins corporels ou de médicaments sur la peau avec un réservoir (1) et un couvercle d'obturation (2) sur le côté intérieur duquel, lorsque le réservoir est fermé, est fixé un dispositif d'application faisant saillie vers l'intérieur,
caractérisé en ce que
le dispositif d'application présente un élément en feutre mince (3), d'une largeur telle qu'il repose
d'une manière amovible par son extrémité dans un logement (5) qui présente la section transversale d'un rectangle plat et qui s'étend verticalement à partir du couvercle d'obuturation (2),
et en ce que
celui-ci est d'une élasticité telle qu'il peut être guidé sans qu'il faille exercer une pression indésirable sur la peau sur une grande superficie de celle-ci, l'élément en feutre étant d'une longueur telle à atteindre lorsque le récipient est fermé, le fond.

2. Applicateur selon la revendication 1, caractérisé en ce que des nervures de limitation de poussée (6) pour l'élément de feutre (3) sont disposées dans le logement (5).

3. Applicateur selon la revendication 1 ou 2, caractérisé en ce que le logement (5) s'étend diamétralement dans le couvercle d'obturation (2).

4. Applicateur selon au moins l'une des revendications 1 à 3, caractérisé en ce que l'élément de filtre (3) a une épaisseur de 3 mm, une largeur de 20 mm et une hauteur de 20 mm.
